(19) Europäisches Patentamt
European Patent Office
Office européen des brevets

(11) EP 3 403 065 B1

(12) **EUROPEAN PATENT SPECIFICATION**

(45) Date of publication and mention of the grant of the patent:
**01.01.2020 Bulletin 2020/01**

(21) Application number: **17700722.6**

(22) Date of filing: **10.01.2017**

(51) Int Cl.:
***G01N 15/02*** *(2006.01)* ***G01N 1/22*** *(2006.01)*
***B01D 1/00*** *(2006.01)* ***B01B 1/00*** *(2006.01)*
***A61K 31/465*** *(2006.01)* ***B01D 45/08*** *(2006.01)*

(86) International application number:
**PCT/GB2017/050047**

(87) International publication number:
**WO 2017/121996 (20.07.2017 Gazette 2017/29)**

(54) **CASCADE IMPACTOR PLATE COATING**

KASKADENIMPAKTORPLATTENBESCHICHTUNG

REVÊTEMENT DE PLAQUE D'IMPACTEUR EN CASCADE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.01.2016 GB 201600641**

(43) Date of publication of application:
**21.11.2018 Bulletin 2018/47**

(73) Proprietor: **Kind Consumer Limited**
**London**
**Greater London EC1R 5AR (GB)**

(72) Inventor: **SILCOCK, Alan James**
**London**
**Greater London WC2R 3LA (GB)**

(74) Representative: **Boult Wade Tennant LLP**
**Salisbury Square House**
**8 Salisbury Square**
**London EC4Y 8AP (GB)**

(56) References cited:
**US-A1- 2005 190 058**
**US-A1- 2007 122 349**
**US-B1- 8 309 029**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 403 065 B1

## Description

### Field

The present invention relates to a cascade impactor and methods of using the same.

### Background

Cascade impactors have been used for a number of years to determine the aerodynamic particle size distribution of aerosol particles. The aerodynamic particle size of inhaled products is important since the particle size directly influences the regional deposition of the aerosol in the lungs and respiratory tract.

Therefore, the measurement of the particle size distribution of aerosol particles is of importance during the development and manufacture of inhaler products which generate aerosols for delivery of a substance to the lungs or respiratory tract of the user. A number of suitable techniques for measuring the particle size of such aerosol particles have been developed. These include, for example, particle time-of-flight spectrometry, laser diffractometry, Phase-Doppler particle size analysis, and inertial techniques such as cascade impaction.

As described in the article "Understanding cascade impaction and its importance for inhaler testing", Mark Copley, www.copleyscientific.com, July 2007, the fraction of a dose that will deposit in a user's lung is defined as the fine particle fraction (FPF). The FPF is dependent on the particle size of the aerosol. The upper limit used to define the FPF varies, but tends to lie between four and six microns, the optimal size for central airway deposition. Peak deposition in the peripheral airways of the lung occurs at between two and four microns. In general, there is no lower limit defined for FPF, although there is an argument for one since very small particles below one micron may be exhaled. The measurement range of interest for inhalation product development is therefore around ten microns and below. Patent application US 2007/0122349 A1 discloses a cascade impactor of the prior art.

The primary advantage offered by inertial techniques compared with the alternative methods described above is that they allow determination of an assay for the FPF and other size fractions. The other methods provide no differentiation between active pharmaceutical ingredients (APIs) and any other components in the formulation; they simply measure the overall particle size distribution.

In addition, inertial techniques are also able to directly measure aerodynamic particle diameter, which is the parameter most closely correlated with particle behaviour during inhalation. Time-of-flight analysers may also measure this variable, but with other techniques it must be calculated from the volume equivalent diameter.

In particular, cascade impactors have been adopted as the standard technique recommended by regulatory bodies for the validation of new inhalation products since they uniquely provide the required degree of resolution in the particle size range of greatest interest for inhalation products: 0.5 to 5 microns.

In some embodiments, the present invention seeks to provide an improved cascade impactor for measuring the particle size distribution of an aerosol comprising nicotine, and a method for using the same.

According to a first aspect to the present invention, there is provided a cascade impactor according to claim 1. According to a second aspect there is a method for measuring the particle size distribution of an aerosol according to claim 11. According to a third aspect there is a use of isopropyl alcohol in solution with heptadecane according to claim 15.

### Brief Description of the Figures

Embodiments of the present invention are described, by way of example only, with reference to the accompanying drawings in which:

Figure 1 shows the principle of operation of a multi-stage cascade impactor.
Figure 2 shows a collection curve efficiency for a sample collection stage in a cascade impactor.
Figure 3 shows an exemplary cascade impactor in accordance with the present invention.
Figure 4A shows a schematic diagram of the orientation of sample collection stages in an NGI (Copley Scientific).
Figure 4B shows a schematic diagram of NGI (Copley Scientific).
Figure 5 shows a schematic diagram of an ACI (Copley Scientific).
Figure 6 shows a schematic diagram of a MMI (Copley Scientific).
Figure 7 shows a schematic diagram of a Twin Impinger (Copley Scientific).

While the invention is susceptible to various modifications and alternative forms, specific embodiments are shown by way of example in the drawings and are herein described in detail. It should be understood, however, that the drawings and detailed description of the specific embodiments are not intended to limit the invention to the particular forms disclosed. On the contrary, the invention covers all modifications falling within the scope of the appended claims.

## Detailed Description

The present invention relates to the cascade impactor. In particular, the present invention relates to a cascade impactor for measuring the particle size distribution of an aerosol comprising nicotine.

Generally, a cascade impactor comprises at least one sample collection stage, each stage having a nozzle assembly and collection means. The collection means typically includes an impaction surface for the collection of the particles.

The principle of operation of cascade impactors is described in the 2015 edition of Copley Scientific, Quality Solutions for Inhaler Testing Brochure. Cascade impactors separate particles based on the principle of inertial impaction. In a cascade impactor, particles in an aerosol are removed from an air stream based on their inertia when the air stream changes direction. Particles above a certain size possess so much momentum that they cannot follow the air stream and thus strike a collection surface which is available for later analysis of mass and composition. The inertia of a particle is a function of particle size and velocity.

In a cascade impactor, sample-laden air is accelerated through an orifice or nozzle towards a collections means at some distance below the nozzle; this causes the air stream to abruptly change direction. Particles which are below a certain size, and therefore have a low enough inertia, remain suspended in the air stream, while larger particles with a higher inertia impact on the collection means. In general, particles larger than the cut size diameter of the impactor impinge or impact on the collection means, where the cut size diameter is the size of particles which are collected with 50% collection efficiency. Collection efficiency increases for particles larger than the cut size diameter, and decreases for smaller particles. The cut size diameter for an impactor is dependent on the diameter of the orifice or nozzle and the flow rate.

Some cascade impactors have multiple collection stages (e.g. two or more) arranged in series, each stage having a nozzle assembly where the nozzle orifice decreases in size relative to that of the previous stage.

During use of a cascade impactor having multiple stages, the sample-laden air is introduced into the impactor and is drawn through the impactor. The air stream flows sequentially through the sample collection stages. At each sample collection stage, the smaller the nozzle orifice, the higher the velocity of air or gas particles moving through the orifice. The higher the velocity, the smaller the particles that are collected on the impaction plate. Therefore, particles of decreasing size may be collected on each sequential sample collection stage.

Particles larger than the cut size diameter of the impactor impinge or impact upon the impaction surface of the collection means. The smaller particles pass with the air stream out of the first impaction region and proceed to the next stage. This procedure continues through the cascade impactor with each stage having higher velocity and collecting smaller size particles.

This principle of operation of a typical multi-stage cascade impactor is shown in Figure 1 (Copley Scientific, Quality Solutions for Inhaler Testing). The sample-laden air stream 3 passes through a nozzle 2 of the cascade impactor 1. The particles in the air stream 3 either subsequently impact on the collection means 4 or are carried along in the air stream which is directed at an angle to the flow-stream through the nozzle. The impaction depends on the inertia of the particles.

As described in the article "Understanding cascade impaction and its importance for inhaler testing", Mark Copley, www.copleyscientific.com, July 2007, ideally collection efficiency would be a step function - all of the particles above a certain size would be captured and those below it would pass through to the next collection stage. However, in practice, there is generally a curve from which the D50 particle size, the cut size diameter of the sample collection stage, is determined. Figure 2 shows a collection efficiency curve for a typical cascade impactor stage as shown in the Copley reference article.

In some instances, particles may "bounce" as opposed to impact when they contact the collection means of a sample collection stage. As used herein, "bounce" refers to the phenomenon where the aerosol particles impact the collection means of a sample collection stage, but subsequently become re-entrained in the air stream rather than remaining on the collection means; i.e. they bounce off the surface of the collection means. The "bounce" of particles leads to them being re-entrained in the sample-laden air stream such that they are carried to a sample collection stage further downstream, where they ultimately impact a sample collection stage having a cut size diameter configured to collect smaller particles..

Previously, this problem of re-entrainment has been addressed by coating the collection means of the sample collection stages with a suitable surface coating, such as glycerin, Tween 80 or silicone oil.

As used herein the term "cascade impactor" includes devices which act as an impactor or as an impinger. As used herein the term "impactor" describes an instrument where the particles impact on a dry impaction collection means. As used herein the term "impinger" describes an instrument where the particles impact on a liquid collection means.

The cascade impactor in accordance with the present invention comprises an induction port for receiving an inhaler and at least one sample collection stage, wherein each sample collection stage comprises a nozzle assembly and a collection means.

"Inhaler" as used herein includes an aerosol-generating means which generates an aerosol comprising nicotine.

A cascade impactor in accordance with the present invention is shown in Figure 3. During use of the cascade impactor 30, an inhaler 31 is received into the induction port 32 of the cascade impactor 30. Upon actuation of the inhaler 31, an aerosol comprising nicotine is produced resulting in a nicotine-laden air stream entering the cascade impactor 30. The nicotine-laden air stream subsequently passes through the at least one collection stage 33.

In some embodiments, a vacuum pump or air pump is attached at the outlet end 34 of the cascade impactor. The outlet end 34 of a cascade impactor is further downstream than the final sample collection stage in the cascade impactor. The vacuum pump or air pump may be used to draw the air stream released from the inhaler through the cascade impactor.

In some embodiments, the cascade impactor comprises at least two sample collection stages. In some embodiments, the cascade impactor comprises at least three sample collection stages. In some embodiments, the cascade impactor comprises at least four sample collection stages. In some embodiments, the cascade impactor comprises at least five sample collection stages. In some embodiments, the cascade impactor comprises at least six sample collection stages. In some embodiments, the cascade impactor comprises at least seven sample collection stages.

In some embodiments, the cascade impactor comprises two sample collection stages. In some embodiments, the cascade impactor comprises three sample collection stages. In some embodiments, the cascade impactor comprises four sample collection stages. In some embodiments, the cascade impactor comprises five sample collection stages. In some embodiments, the cascade impactor comprises six sample collection stages. In some embodiments, the cascade impactor comprises seven sample collection stages. In some embodiments, the cascade impactor comprises eight sample collection stages.

In some embodiments, the cascade impactor further comprises an internal filter or an external filter. Such filters may be used to collect ultra-fine particles that are not impacted on any of the sample collection stages in the cascade impactor. This may be useful, for example, where the particle size of the aerosol particles is less than the cut size diameter of the sample collection stage having the smallest cut size diameter (i.e. the sample collection stage furthest downstream in the cascade impactor.

In some embodiments the cascade impactor is selected from a Next Generation Impactor™ (NGI), an Anderson Cascade Impactor™ (ACI), a Multi-stage Liquid Impinger™(MSLI), a Marple-Miller Cascade Impactor™(MMI) and a Twin Impinger™.

In some embodiments, the cascade impactor is an NGI. A "Next Generation Impactor" as used herein includes a cascade impactor that comprises seven sample collection stages. In some embodiments, the sample collection stages are arranged in a planar configuration. The NGI may include a horizontal tray for receiving each of the sample collection stages. The sample collection stages may each comprise a nozzle assembly, and a collection means in the form of a cup.

In some embodiments, the NGI operates at a flow rate of between 30 and 100 L/min. The particle size range measurable by an NGI may be between 0.24 to 11.7μm depending on the flow rate of the NGI.

In some embodiments, the NGI comprises seven sample collection stages and a micro-orifice collector (MOC). The MOC may comprise a nozzle assembly such that any particles remaining in the air stream after passing through the seventh sample collection stage may be impacted on the MOC. The MOC may thus be able to collect all remaining particles having a particle size below the cut size diameter of the seventh collection stage. This eliminates the need for a final filter paper to be present downstream of the seventh collection stage.

In some embodiments, the NGI comprises a cup tray containing the seven sample collection stage in the form of cups, and the MOC. The cups may be used to collect the samples prior to analysis. In some embodiments, the NGI comprises a frame to support the cup tray. In some embodiments, the NGI comprises a lid containing the inter-stage pathways for directing the air stream between the sample collection stages. In some embodiments, the lid holds the nozzle assemblies of each sample collection stage in place.

A schematic of an exemplary NGI as marketed by Copley Scientific is shown in Figures 4A and 4B. As shown in Figure 4A, the NGI comprises seven sample collection stages and an MOC. The stages are arranged such that the air stream passing through the impactor travels in a saw tooth pattern. The NGI 40 shown in Figure 4B comprises a lid 45 with a seal body attached for holding the nozzle assemblies 41 of each sample collection stage in place. The lid 45 is hingedly attached to a frame 42 comprising a removable cup tray 43 which houses eight cups 44 as collection means for each sample collection stage and the MOC. The inter-stage passageway 46 of the air stream is shown as a dotted line in Figure 4B.

In some embodiments, the cascade impactor is an NGI as produced by Copley Scientific (see Figures 4A and 4B). In this embodiment, the cut size diameters of each of the sample collection stages in the NGI are as follows:

| Cut Size Diameter at flow rate of: | 15 L/min | 30 L/min | 60 L/min | 100 L/min |
|---|---|---|---|---|
| **Stage** 1 | 14.10 μm | 11.76 μm | 8.06 μm | 6.12 μm |

(continued)

| Cut Size Diameter at flow rate of: | 15 L/min | 30 L/min | 60 L/min | 100 L/min |
|---|---|---|---|---|
| **Stage 2** | 8.61 μm | 6.40 μm | 4.46 μm | 3.42 μm |
| **Stage 3** | 5.39 μm | 3.99 μm | 2.82 μm | 2.18 μm |
| **Stage 4** | 3.30 μm | 2.30 μm | 1.66 μm | 1.31 μm |
| **Stage 5** | 2.08 μm | 1.36 μm | 0.94 μm | 0.72 μm |
| **Stage 6** | 1.36 μm | 0.83 μm | 0.55 μm | 0.40 μm |
| **Stage 7** | 0.98 μm | 0.54 μm | 0.34 μm | 0.24 μm |
| **MOC** | 0.70 μm | 0.36 μm | 0.14 μm | 0.07 μm |

In other words, there are seven sample collection stages in the Copley Scientific NGI, five with cut size diameters between 0.54 and 6.12 μm at flow rates from 30 to 100 L/min.

As shown in Figure 4B, the sample aerosol air stream passes through the impactor in a saw tooth pattern. Particle separation and size measurement may be achieved by successively increasing the velocity of the air stream as it passes through each sample collection stage by forcing it through a series of nozzles containing progressively reducing jet diameters.

In some embodiments, the cascade impactor is an ACI. An "Andersen Cascade Impactor" as used herein includes a cascade impactor that comprises eight sample collection stages that are typically arranged in a stacked design. The stacked design of an ACI makes for easy handling, and damaged stages can simply be removed and replaced when necessary.

In some embodiments, the ACI operates at a flow rate between 28.3 and 100 L/min. In some embodiments, the ACI operates at a flow rate of 60 L/min. In some embodiments, the ACI operates at a flow rate of 90 L/min.

A schematic of an exemplary ACI attached to a vacuum pump as marketed by Copley Scientific is shown in Figure 5. The ACI 50 comprises eight sample collection stages 51 arranged in a stack, one on top of the other. An induction port 52 is positioned on top of the first sample collection stage such that when an inhaler 53 is attached to the induction port, and an aerosol released from the inhaler, the aerosol air stream flows through the sample collection stages sequentially from the first stage to the eighth stage. The vacuum pump 54 is used to draw the aerosol air stream through the ACI.

In some embodiments, the ACI has eight sample collection stages: stage 0, stage 1, stage 2, stage 3, stage 4, stage 5, stage 6, and stage 7. In some embodiments, stage 0 has a cut size diameter such that it collects particles with particle sizes of at least 9 μm. In some embodiments, stage 1 has a cut size diameter such that it collects particles with particle sizes of from 5.8 to 9 μm. In some embodiments, stage 2 has a cut size diameter such that it collects particles with particle sizes of from 4.7 to 5.8 μm. In some embodiments, stage 3 has a cut size diameter such that it collects particles with particle sizes of from 3.3 to 4.7 μm. In some embodiments, stage 4 has a cut size diameter such that it collects particles with particle sizes of from 2.1 to 3.3 μm. In some embodiments, stage 5 has a cut size diameter such that it collects particles with particle sizes of from 1.1 to 2.1 μm. In some embodiments, stage 6 has a cut size diameter such that it collects particles with particle sizes of from 0.7 to 1.1 μm. In some embodiments, stage 7 has a cut size diameter such that it collects particles with particle sizes of from 0.4 to 0.7 μm.

In some embodiments, the cascade impactor is an MSLI. A "Multi-stage Liquid Impinger" as used herein includes a cascade impactor (or liquid impinger) comprising five sample collection stages which can be used for determining the particle size (aerodynamic size distribution) of inhalers. An MSLI may typically have the sample collection stages arranged in a stacked design, similar to the ACI. The sample collection stages of the MSLI are typically kept moist in order to reduce the problem of re-entrainment.

In some embodiments, the MSLI operates at a flow rate between 30 and 100 L/min. In some embodiments, the cascade impactor is an MSLI marketed by Copley Scientific. The Copley Scientific MSLI comprises four sample collection stages having cut size diameters decreasing in increments from 13 to 1.7 microns; the fifth stage comprises an integral paper filter to capture the remaining fraction of particles having a D50 particle size of less than 1.7 microns.

In some embodiments, the cascade impactor is an MMI. A "Marple-Miller Cascade Impactor" as used herein is a cascade impactor including five sample collection stages and an induction port. In some embodiments, the collection means of the sample collection stages are in the form of cups for collecting the aerosol particles.

In some embodiments, the MMI operates at a flow rate of between 4.9 and 90 L/min. In some embodiments, the MMI operates at a flow rate between 60 and 90 L/min (Model 160). In some embodiments, the MMI operates at a flow rate between 30 and 60 L/min (Model 150). In some embodiments, the MMI operates at a flow rate between 4.9

and 12 L/min (Model 150P).

In some embodiments, the MMI comprises a paper filter downstream of the fifth sample collection stage in order to ensure that all aerosol particles of are collected in the impactor.

The cut size diameter of each sample collection stage in an MMI may be calculated using the following equation:

$$D50(Q) = D50(Q_n)\left(\frac{Q_n}{Q}\right)^{1/2}$$

where D50(Q) is the cut size diameter of the sample collection stage at the flow rate Q, $Q_n$ is a flow rate of 60 L/min, and D50($Q_n$) is the cut size diameter of the sample collection stage at a flow rate of 60 L/min.

A schematic diagram of an exemplary MMI marketed by Copley Scientific is shown in Figure 6. The MMI 60 comprises five sample collection stages 61 in the form of cups, and an induction port 62 for receiving an inhaler. A filter 63 is also included downstream of the fifth sample collection stage. A port at the outlet end 64 enables connection of the cascade impactor to a vacuum pump or air pump for drawing the aerosol from the inhaler through the MMI.

In some embodiments, the cascade impactor is an MMI marketed by Copley Scientific. The cut size diameters of each of the five sample collection stages at a flow rate of 60 L/min (Model 160) are as follows:

Stage 1 - 10 μm
Stage 2 - 5 μm
Stage 3 - 2.5 μm
Stage 4 - 1.25 μm
Stage 5 - 0.63 μm.

In some embodiments, the cascade impactor is a Twin Impinger. A "Twin Impinger" as used herein includes a cascade impactor comprising two sample collection stages. A schematic diagram of an exemplary Twin Impinger is shown in Figure 7. The Twin Impinger 70 comprises a first stage 71 which is configured to collect aerosol particles that have a particle size such that they would impact on the oropharynx of the user inhaling the aerosol. The Twin Impinger 70 also comprises a second stage 72 which is configured to collect aerosol particles that would penetrate the lungs of a user inhaling the aerosol.

In some embodiments, the cut size diameter of the first stage 71 is 6.4 μm. Particles smaller than 6.4 μm may pass through to the second stage 72.

In some embodiments, the outlet end 73 of the Twin Impinger is configured to be attached to a vacuum pump such that the aerosol is drawn through the Twin Impinger.

In accordance with the present invention, at least one sample collection stage of the cascade impactor is coated with isopropyl alcohol in solution with heptadecane.

"Isopropyl alcohol" as used herein is a compound with the chemical formula $C_3H_8O$ and the following chemical structure:

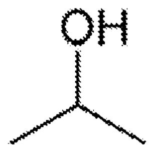

Isopropyl alcohol may also be referred to as isopropanol or 2-propanol. Isopropyl alcohol is a colourless, flammable chemical compound with a strong odour, and has a wide variety of industrial and household uses.

In accordance with the present invention, at least one sample collection stage in the cascade impactor is coated with isopropyl alcohol in solution with heptadecane. In some embodiments where there is more than one sample collection stage in the cascade impactor, at least two sample collection stages are coated with isopropyl alcohol in solution with heptadecane, such as at least three sample collection stages are coated with isopropyl alcohol in solution with heptadecane, such as at least four sample collection stages are coated with isopropyl alcohol in solution with heptadecane, such as at least five sample collection stages are coated with isopropyl alcohol in solution with heptadecane, such as at least six sample collection stages are coated with isopropyl alcohol in solution with heptadecane, such as at least seven sample collection stages are coated with isopropyl alcohol in solution with heptadecane.

In some embodiments, each of the sample collection stages in the cascade impactor is coated with isopropyl alcohol in solution with heptadecane.

In some embodiments, the cascade impactor is an NGI. In some embodiments, the NGI comprises seven

sample collection stages. In some embodiments, the NGI comprises seven sample collection stages and an MOC. In some embodiments, at least one of the sample collection stages in the NGI is coated with isopropyl alcohol in solution with heptadecane. In some embodiments, at least two of the sample collection stages in the NGI are coated with isopropyl alcohol in solution with heptadecane. In some embodiments, at least three of the sample collection stages in the NGI are coated with isopropyl alcohol in solution with heptadecane. In some embodiments, at least four of the sample collection stages in the NGI are coated with isopropyl alcohol in solution with heptadecane. In some embodiments, at least five of the sample collection stages in the NGI are coated with isopropyl alcohol in solution with heptadecane. In some embodiments, at least six of the sample collection stages in the NGI are coated with isopropyl alcohol in solution with heptadecane. In some embodiments, all seven of the sample collection stages in the NGI are coated with isopropyl alcohol in solution with heptadecane. In some embodiments, the MOC is not coated with isopropyl alcohol in solution with heptadecane. In some embodiments, the MOC is coated with isopropyl alcohol in solution with heptadecane.

In some embodiments, the cascade impactor is an ACI. In some embodiments, the ACI comprises eight sample collection stages. In some embodiments, at least one of the sample collection stages in the ACI is coated with isopropyl alcohol in solution with heptadecane. In some embodiments, at least two of the sample collection stages in the ACI are coated with isopropyl alcohol in solution with heptadecane. In some embodiments, at least three of the sample collection stages in the ACI are coated with isopropyl alcohol in solution with heptadecane. In some embodiments, at least four of the sample collection stages in the ACI are coated with isopropyl alcohol in solution with heptadecane. In some embodiments, at least five of the sample collection stages in the ACI are coated with isopropyl alcohol in solution with heptadecane. In some embodiments, at least six of the sample collection stages in the ACI are coated with isopropyl alcohol in solution with heptadecane. In some embodiments, at least seven of the sample collection stages in the ACI are coated with isopropyl alcohol in solution with heptadecane. In some embodiments, all eight of the sample collection stages in the ACI are coated with isopropyl alcohol in solution with heptadecane.

In some embodiments, the isopropyl alcohol is present in an amount of no greater than 5 mL in each of the sample collection stages that are coated with isopropyl alcohol in solution with heptadecane, such as in an amount of no greater than 4 mL in each of the sample collection stages that are coated with isopropyl alcohol in solution with heptadecane, such as in an amount of no greater than 3 mL in each of the sample collection stages that are coated with isopropyl alcohol in solution with heptadecane.

According to the present invention, the isopropyl alcohol is present in solution with heptadecane.

In some embodiments, the concentration of heptadecane in isopropyl alcohol is from 10 μg/mL to 50 μg/mL, such as from 10 μg/mL to 40 μg/mL, such as from 15 μg/mL to 40 μg/mL, such as from 15 μg/mL to 30 μg/mL, such as from 15 μg/mL to 25 μg/mL, such as about 20 μg/mL.

The use of small amounts of isopropyl alcohol in solution with heptadecane in the sample collection stages may be advantageous since such small amounts do not significantly reduce the distance between the nozzle assembly and the collection means in the sample collection stage. Therefore, the presence of the isopropyl alcohol in solution with heptadecane coating may not significantly affect the impaction properties of the aerosol particles on the collection means.

An advantage of the present invention is that the extent of evaporation of the nicotine collected on the sample collection stage(s) may be reduced such that the collection efficiency and measurement accuracy of the impactor may be increased. Nicotine is a readily volatile chemical compound, which has a vapour pressure of 5.5 Pa at 25°C. The inventors thus found that, when using a cascade impactor to measure the particle size distribution of an aerosol comprising nicotine, the degree of accuracy and the collection efficiency was lower than for other aerosol substances. Without wishing to be bound, this may be attributed to the nicotine impacting on the sample collection stage(s) being evaporated and subsequently being lost into the vacuum pump at the end of the impactor, or being deposited at a collection stage further down the impactor (i.e. a sample collection stage with a smaller cut size diameter than the D50 particle size of the nicotine particle).

The use of the isopropyl alcohol in solution with heptadecane in the present invention advantageously decreases the extent of evaporation of the nicotine from the sample collection stages, and thus increases the proportion of nicotine particles which remain impacted on the sample collection stage having the appropriate cut size for the size of the nicotine particles. This results in a higher degree of accuracy of the cascade impactor when measuring the particle size distribution of an aerosol comprising nicotine.

Therefore, in one embodiment, there is provided use of isopropyl alcohol in solution with heptadecane for reducing the extent of evaporation of nicotine during use of a cascade impactor for measuring the particle size distribution of an aerosol comprising nicotine.

In some embodiments, at least one sample collection stage of the cascade impactor is coated with glycerin, silicone oil, Tween 80 or a mixture thereof.

The coating of the sample collection stages with any one or more of these components may advantageously reduce the bounce of the particles off the collection means of the sample collection stage, thus increasing the collection efficiency of the cascade impactor.

In some embodiments where there is more than one sample collection stage in the cascade impactor, at least

two sample collection stages are coated with glycerin, silicone oil, Tween 80 or a mixture thereof, such as at least three sample collection stages are coated with glycerin, silicone oil, Tween 80 or a mixture thereof, such as at least four sample collection stages are coated with glycerin, silicone oil, Tween 80 or a mixture thereof, such as at least five sample collection stages are coated with glycerin, silicone oil, Tween 80 or a mixture thereof, such as at least six sample collection stages are coated with glycerin, silicone oil, Tween 80 or a mixture thereof, such as at least seven sample collection stages are coated with glycerin, silicone oil, Tween 80 or a mixture thereof.

In some embodiments, each of the sample collection stages present in the cascade impactor are coated with glycerin, silicone oil, Tween 80 or a mixture thereof.

In some embodiments, the cascade impactor is an NGI. In some embodiments, the NGI comprises seven sample collection stages. In some embodiments, the NGI comprises seven sample collection stages and an MOC. In some embodiments, at least one of the sample collection stages in the NGI is coated with glycerin, silicone oil, Tween 80 or a mixture thereof. In some embodiments, at least two of the sample collection stages in the NGI are coated with glycerin, silicone oil, Tween 80 or a mixture thereof. In some embodiments, at least three of the sample collection stages in the NGI are coated with glycerin, silicone oil, Tween 80 or a mixture thereof. In some embodiments, at least four of the sample collection stages in the NGI are coated with glycerin, silicone oil, Tween 80 or a mixture thereof. In some embodiments, at least five of the sample collection stages in the NGI are coated with glycerin, silicone oil, Tween 80 or a mixture thereof. In some embodiments, at least six of the sample collection stages in the NGI are coated with glycerin, silicone oil, Tween 80 or a mixture thereof. In some embodiments, all seven of the sample collection stages in the NGI are coated with glycerin, silicone oil, Tween 80 or a mixture thereof. In some embodiments, the MOC is not coated with glycerin, silicone oil, Tween 80 or a mixture thereof. In some embodiments, the MOC is coated with glycerin, silicone oil, Tween 80 or a mixture thereof.

The present invention also provides a method for measuring the particle size distribution of an aerosol comprising nicotine according to claim 14.

In some embodiments the method further comprises the steps of:

(c) removing the sample collection stages from the cascade impactor; and
(d) analysing the sample collected on each sample collection stage to determine the particle size distribution of said sample.

In some embodiments, the analysing step (d) comprises a chromatographic method. In some embodiments, the analysing step (d) comprises high pressure liquid chromatography of the sample collected on each sample collection stage.

In some embodiments, the inhaler is attached to cascade impactor by inserting the inhaler into the induction port of the cascade impactor. The inhaler may subsequently be actuated to dispense the aerosol comprising nicotine into the cascade impactor, where the particle size distribution of the aerosol comprising nicotine may then be measured.

**Examples**

The invention will now be described with reference to the following non-limiting example. A cascade impactor in accordance with the present invention was set up in order to determine the particle size distribution of an aerosol comprising nicotine.

The apparatus was set up as detailed below. The experiment was conducted using a Copley Scientific NGI, as described in detail above, and as shown in Figures 4A and 4B. An isopropyl alcohol solution was added to the sample collection stages of the NGI, and an aerosol comprising nicotine introduced into the device. Subsequently, the samples collected on each stage in the NGI were collected and analysed using gas chromatography.

Diluent Preparation

A 1 mg/mL stock solution of heptadecane in IPA was prepared. 100 mg heptadecane was weighed into a 100 mL volumetric flask. The volumetric flask was made up to volume using IPA.

A 1 L solution of heptadecane in IPA (20 μg/mL) was prepared. 20 mL heptadecane was diluted in IPA (1 mg/mL) in a volumetric flask, and then made up to volume using IPA. This solution was stable for 1 month.

Preparation of nicotine stock solutions

100 mg (± 1 mg) nicotine was weighed and transferred to a 200 mL volumetric flask, which was made up to volume using IPA. This stock solution was prepared in duplicate.

Flow Adapter Preparation

A Dose Unit Sampling Apparatus (DUSA) was assembled as per the following steps, and the airflow through the DUSA adjusted to 30 L/min:

i) A vacuum pump was connected to a Copley TPK (a critical flow controller valve that has a timed solenoid valve). The other side of the TPK was connected to the filter holder of a DUSA. With the filter holder clamped in a retort stand in the vertical position, a Type A/E Glass Fibre filter (Pall Corp) was placed on the filter support, and a DUSA tube attached to hold the filter in place. The DUA assembly was rotated so that the DUSA tube was in the horizontal orientation.
ii) An unused inhaler device was inserted into the mouthpiece of a Flow Adapter, and the needle valve was fully closed.
iii) The Flow Adapter was attached to the DUSA using the associated sleeve of the Flow Adapter, and the air flow was started using the TPK.
iv) A flow meter (Copley Scientific model DFM3) was attached to the orifice of the Flow Adapter, and the needle valve slowly opened until the flow reached the desired value of 23 L/min (where 23 L/mine equates to the desired flow rate into the NGI of 30 L/min, minus the desired flow rate through the inhaler device of 7 L/min.

NGI Preparation

i) The NGI was assembled and an induction port inserted into the inlet of the NGI. A flow meter was attached to the induction port using a suitable adapter (e.g. Oxette adapter).
ii) The air pump was turned on for 5 minutes to allow the apparatus to warm up prior to testing.
iii) The air flow was started using the TPK, and the flow rate adjusted to 30 L/min (±0.2 L/min).
iv) The air flow was stopped, and the flow meter and induction port removed.
v) The solution of heptadecane in IPA (as formulated under "Diluent Preparation") was added to the cups in quantities as provided in Table 1.
vi) The prepared Flow Adapter was attached to the induction port, ensuring that the throat and Flow Adapter surfaces were flush.

Table 1

| **Stage** | **1** | **2** | **3** | **4** | **5** | **6** | **7** | **MOC** |
|---|---|---|---|---|---|---|---|---|
| **Volume (mL)** | 3.0 | 1.5 | 1.5 | 1.5 | 1.5 | 1.5 | 1.0 | 0.0 |

Device Actuation

i) The inhaler device was filled with aerosol formulation.
ii) The inhaler device was attached to the Flow Adapter.
iii) The air flow was started using the TPK.
iv) The device was removed from the Flow Adapter and weighed.
v) The above steps were repeated so that 2 doses were fired into the NGI.

Sample Recovery

Each section of the NGI was disassembled and washed with a defined volume of diluent (shown in Table 2) to recover the nicotine. A disposable syringe was used to draw sample from the wash volume. The solution was put into vials and analysed using Gas Chromatography with Flame Ionisation Detector (GC-FID).

Table 2

| Stage | Oxette adapter | Throat | Stage 1 | Stage 2 | Stage 3 | Stage 4 | Stage 5 | Stage 6 | Stage 7 | MOC |
|---|---|---|---|---|---|---|---|---|---|---|
| Wash volume (mL) | 10 | 10 | 7 | 9 | 9 | 9 | 9 | 9 | 9 | 10 |

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures and/or other aspects described herein are not to be considered limitations on the scope of the invention as defined by the claims or limitations on equivalents to the claims, and that other embodiments may be utilised and modifications may be made without departing from the scope of the claims. Various embodiments of the invention may suitably comprise, consist of, or consist essentially of appropriate combinations of the disclosed elements, components, features, parts, steps, means, etc., other than those specifically described herein. In addition, this disclosure may include other inventions not presently claimed, but which may be claimed in the future.

## Claims

**1.** A cascade impactor (30, 40, 50, 60, 70) for measuring particle size distribution of an aerosol comprising nicotine, the cascade impactor comprising:

an induction port (32, 52, 62) for receiving an inhaler (31, 53); and
a plurality of sample collection stages (33, 4, 61) each comprising a nozzle assembly and a collection means in the form of an impaction surface for collection of particles from the respective nozzle;
**characterized in that** at least one impaction surface comprises a coating of isopropyl alcohol present in solution with heptadecane.

**2.** A cascade impactor according to claim 1, wherein the cascade impactor (30, 40, 50, 60, 70) comprises at least five collection stages.

**3.** A cascade impactor according to claim 1 or 2, wherein the cascade impactor (30, 40, 50, 60, 70) comprises seven collection stages.

**4.** A cascade impactor according to claim 3, wherein the cascade impactor further comprises a micro-orifice collector.

**5.** A cascade impactor according to any one of claims 1 to 4, wherein the isopropyl alcohol in solution with heptadecane is present in an amount of no greater than 5 mL in each of the sample collection stages that are coated with isopropyl alcohol in solution with heptadecane.

**6.** A cascade impactor according to any one of claims 1 to 5, wherein the isopropyl alcohol in solution with heptadecane is present in an amount of no greater than 3 mL in each of the sample collection stages that are coated with isopropyl alcohol in solution with heptadecane.

**7.** A cascade impactor according to any one of claims 1 to 6, wherein each of the sample collection stages comprises a coating of isopropyl alcohol in solution with heptadecane.

**8.** A cascade impactor according to claim 3 or 4, wherein each of the seven sample collection stages comprises a coating of isopropyl alcohol in solution with heptadecane.

**9.** A cascade impactor according to any one of claims 1 to 8, wherein at least one sample collection stage is coated with glycerin, silicone oil, Tween 80 or a mixture thereof.

**10.** A cascade impactor according to claim 8, wherein each of the seven sample collection stages is also coated with glycerin, silicone oil, Tween 80 or a mixture thereof.

**11.** A method for measuring the particle size distribution of an aerosol comprising nicotine, the method comprising the steps of:

(a) attaching an inhaler for generating the aerosol comprising nicotine to a cascade impactor (30, 40, 50, 60, 70), wherein the cascade impactor comprises:
a plurality of sample collection stages (33, 44, 61) each comprising a nozzle assembly and a collection means in the form of an impaction surface for collection of particles from the respective nozzle; wherein at least one impaction surface comprises a coating of isopropyl alcohol present in solution with heptadecane ;
and
(b) dispensing an aerosol comprising nicotine from the inhaler into the cascade impactor.

**12.** A method according to claim 11 further comprising the steps of:

(c) removing the sample collection stages from the cascade impactor; and
(d) analysing the sample collected on each sample collection stage to determine the particle size distribution of said sample.

**13.** A method according to claim 12, wherein the analysing step (d) comprises gas chromatography.

**14.** A method according to any one of claims 11-13, wherein the cascade impactor is as defined in any one of claims 1 to 10.

**15.** Use of isopropyl alcohol present in solution with heptadecane as an impaction surface coating for reducing the extent of evaporation of nicotine during use of a cascade impactor for measuring the particle size distribution of an aerosol comprising nicotine.

## Patentansprüche

**1.** Kaskadenimpaktor (30, 40, 50, 60, 70) zum Messen einer Partikelgrößenverteilung eines Nikotin aufweisenden Aerosols, wobei der Kaskadenimpaktor aufweist:

eine Einführöffnung (32, 52, 62) zur Aufnahme eines Inhalators (31, 53); und
eine Mehrzahl von Probensammelstufen (33, 4, 61), die jeweils eine Düsenanordnung und ein Sammelmittel in der Form einer Impaktionsoberfläche zum Sammeln von Partikeln von der jeweiligen Düse aufweisen;
**dadurch gekennzeichnet, dass** zumindest eine Impaktionsoberfläche eine Beschichtung von in Lösung mit Heptadekan befindlichem Isopropyl-Alkohol aufweist.

**2.** Kaskadenimpaktor nach Anspruch 1, wobei der Kaskadenimpaktor (30, 40, 50, 60, 70) zumindest fünf Sammelstufen aufweist.

**3.** Kaskadenimpaktor nach Anspruch 1 oder 2, wobei der Kaskadenimpaktor (30, 40, 50, 60, 70) sieben Sammelstufen aufweist.

**4.** Kaskadenimpaktor nach Anspruch 3, wobei der Kaskadenimpaktor ferner einen Mikroöffnungssammler aufweist.

**5.** Kaskadenimpaktor nach einem der Ansprüche 1 bis 4, wobei der Isopropyl-Alkohol in Lösung mit Heptadekan in einer Menge von nicht mehr als 5ml in jeder der Probensammelstufen, die mit Isopropyl-Alkohol in Lösung mit Heptadekan beschichtet sind, vorhanden ist.

**6.** Kaskadenimpaktor nach einem der Ansprüche 1 bis 5, wobei der Isopropyl-Alkohol in Lösung mit Heptadekan in einer Menge von nicht mehr als 3ml in jeder der Probensammelstufen, die mit Isopropyl-Alkohol in Lösung mit Heptadekan beschichtet sind, vorhanden ist.

**7.** Kaskadenimpaktor nach einem der Ansprüche 1 bis 6, wobei jede der Probensammelstufen eine Beschichtung von Isopropyl-Alkohol in Lösung mit Heptadekan aufweist.

**8.** Kaskadenimpaktor nach Anspruch 3 oder 4, wobei jede der sieben Probensammelstufen eine Beschichtung von

Isopropyl-Alkohol in Lösung mit Heptadekan aufweist.

**9.** Kaskadenimpaktor nach einem der Ansprüche 1 bis 8, wobei zumindest eine Probensammelstufe mit Glycerin, Silikonöl, Tween 80 oder einem Gemisch davon beschichtet ist.

**10.** Kaskadenimpaktor nach Anspruch 8, wobei jede der sieben Probensammelstufen auch mit Glycerin, Silikonöl, Tween 80 oder einem Gemisch davon beschichtet ist.

**11.** Verfahren zum Messen der Partikelgrößenverteilung eines Nikotin aufweisenden Aerosols, wobei das Verfahren die Schritte aufweist:

(a) Anbringen eines Inhalators zum Erzeugen des Nikotin aufweisenden Aerosols an einem Kaskadenimpaktor (30, 40, 50, 60, 70), wobei der Kaskadenimpaktor aufweist:
eine Mehrzahl von Probensammelstufen (33, 44, 61), die jeweils eine Düsenanordnung und ein Sammelmittel in der Form einer Impaktionsoberfläche zum Sammeln von Partikeln von der jeweiligen Düse aufweisen; wobei zumindest eine Impaktionsoberfläche eine Beschichtung von in Lösung mit Heptadekan befindlichem Isopropyl-Alkohol aufweist; und
(b) Spenden eines Nikotin aufweisenden Aerosols von dem Inhalator in den Kaskadenimpaktor.

**12.** Verfahren nach Anspruch 11, das ferner die Schritte aufweist:

(c) Entfernen der Probensammelstufen von dem Kaskadenimpaktor; und
(d) Analysieren der in jeder Probensammelstufe gesammelten Probe zur Bestimmung der Partikelgrößenverteilung der Probe.

**13.** Verfahren nach Anspruch 12, wobei der Analysierschritt (d) GasChromatographie aufweist.

**14.** Verfahren nach einem der Ansprüche 11 bis 13, wobei der Kaskadenimpaktor wie in einem der Ansprüche 1 bis 10 definiert ist.

**15.** Verwendung von in Lösung mit Heptadekan befindlichem Isopropyl-Alkohol als Impaktionsoberflächenbeschichtung zum Reduzieren des Ausmaßes der Verdunstung von Nikotin während der Verwendung eines Kaskadenimpaktors zum Messen der Partikelgrößenverteilung eines Nikotin aufweisenden Aerosols.

## Revendications

**1.** Impacteur à cascade (30, 40, 50, 60, 70) pour mesurer la distribution granulométrique d’un aérosol comprenant de la nicotine, l’impacteur à cascade comprenant :

un port d’admission (32, 52, 62) destiné à recevoir un inhalateur (31, 53) ; et
une pluralité d’étages de collecte d’échantillons (33, 4, 61) comprenant chacun un ensemble buse et un moyen de collecte sous la forme d’une surface d’impact pour la collecte de particules à partir de la buse respective ;
**caractérisé en ce qu’**au moins une surface d’impact comprend un revêtement d’alcool isopropylique présent en solution avec de l’heptadécane.

**2.** Impacteur à cascade selon la revendication 1, l’impacteur à cascade (30, 40, 50, 60, 70) comprenant au moins cinq étages de collecte.

**3.** Impacteur à cascade selon la revendication 1 ou 2, l’impacteur à cascade (30, 40, 50, 60, 70) comprenant sept étages de collecte.

**4.** Impacteur à cascade selon la revendication 3, l’impacteur à cascade comprenant en outre un collecteur à micro-orifices.

**5.** Impacteur à cascade selon l’une quelconque des revendications 1 à 4, dans lequel l’alcool isopropylique en solution avec de l’heptadécane est présent en une quantité ne dépassant pas 5 ml dans chacun des étages de collecte d’échantillons qui sont revêtus d’alcool isopropylique en solution avec de l’heptadécane.

**6.** Impacteur à cascade selon l'une quelconque des revendications 1 à 5, dans lequel l'alcool isopropylique en solution avec de l'heptadécane est présent en une quantité ne dépassant pas 3 ml dans chacun des étages de collecte d'échantillons qui sont revêtus d'alcool isopropylique en solution avec de l'heptadécane.

**7.** Impacteur à cascade selon l'une quelconque des revendications 1 à 6, dans lequel chacun des étages de collecte d'échantillons comprend un revêtement d'alcool isopropylique en solution avec de l'heptadécane.

**8.** Impacteur à cascade selon la revendication 3 ou 4, dans lequel chacun des sept étages de collecte d'échantillons comprend un revêtement d'alcool isopropylique en solution avec de l'heptadécane.

**9.** Impacteur à cascade selon l'une quelconque des revendications 1 à 8, dans lequel au moins un étage de collecte d'échantillons est revêtu de glycérine, d'huile de silicone, de Tween 80 ou d'un mélange de ceux-ci.

**10.** Impacteur à cascade selon la revendication 8, dans lequel chacun des sept étages de collecte d'échantillons est également revêtu de glycérine, d'huile de silicone, de Tween 80 ou d'un mélange de ceux-ci.

**11.** Procédé de mesure de la distribution granulométrique d'un aérosol comprenant de la nicotine, le procédé comprenant les étapes consistant à :

(a) fixer un inhalateur destiné à générer l'aérosol comprenant de la nicotine à un impacteur à cascade (30, 40, 50, 60, 70), où l'impacteur à cascade comprend :

une pluralité d'étages de collecte d'échantillons (33, 44, 61) comprenant chacun un ensemble buse et un moyen de collecte sous la forme d'une surface d'impact pour la collecte de particules à partir de la buse respective ;
où au moins une surface d'impact comprend un revêtement d'alcool isopropylique présent en solution avec de l'heptadécane ; et

(b) distribuer un aérosol comprenant de la nicotine à partir de l'inhalateur dans l'impacteur à cascade.

**12.** Procédé selon la revendication 11, comprenant en outre les étapes consistant à :

(c) retirer les étages de collecte d'échantillons de l'impacteur à cascade ; et
(d) analyser l'échantillon collecté dans chaque étage de collecte d'échantillons pour déterminer la distribution granulométrique dudit échantillon.

**13.** Procédé selon la revendication 12, dans lequel l'étape d'analyse (d) comprend une chromatographie en phase gazeuse.

**14.** Procédé selon l'une quelconque des revendications 11 à 13, dans lequel l'impacteur à cascade est tel que défini dans l'une quelconque des revendications 1 à 10.

**15.** Utilisation d'alcool isopropylique présent en solution avec de l'heptadécane en tant que revêtement de surface d'impact pour réduire le degré d'avancement de l'évaporation de la nicotine lors de l'utilisation d'un impacteur à cascade pour mesurer la distribution granulométrique d'un aérosol comprenant de la nicotine.

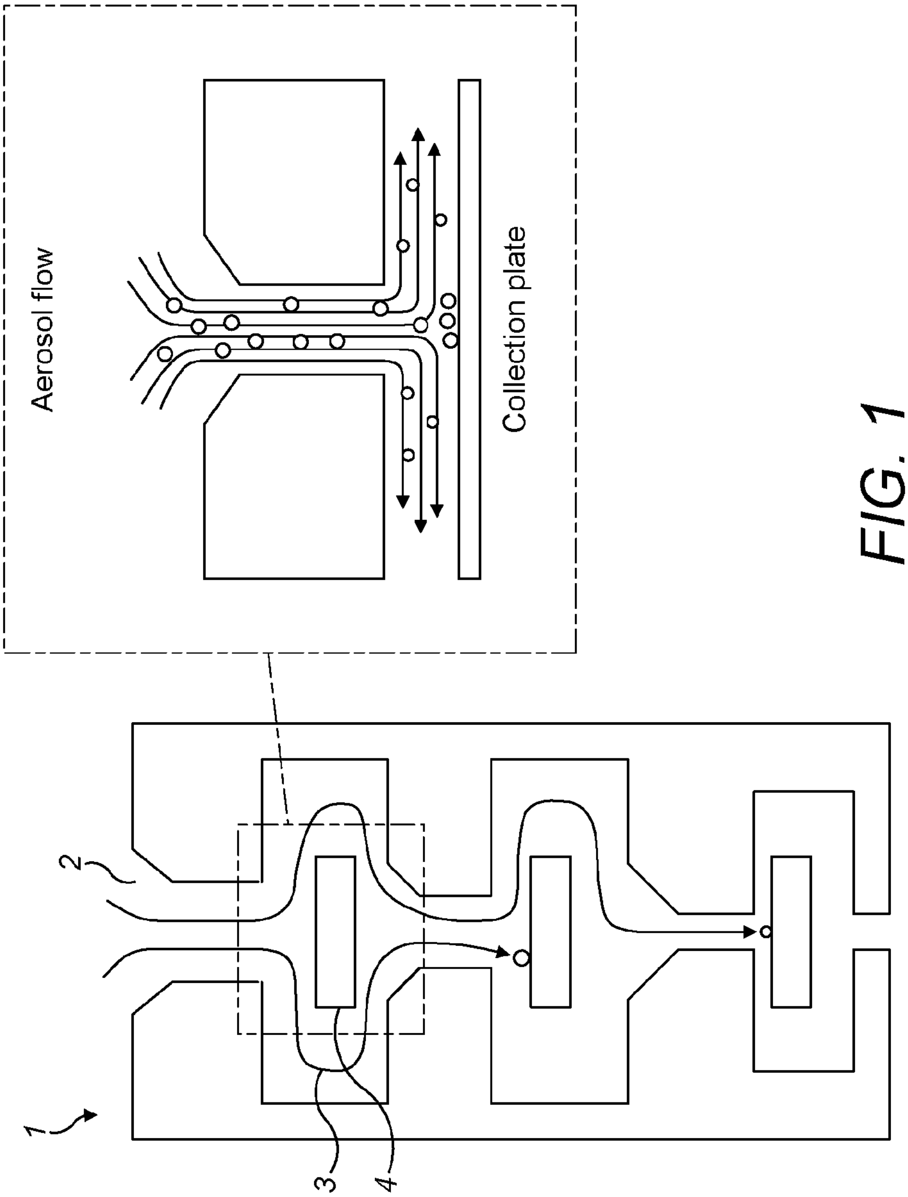


FIG. 1

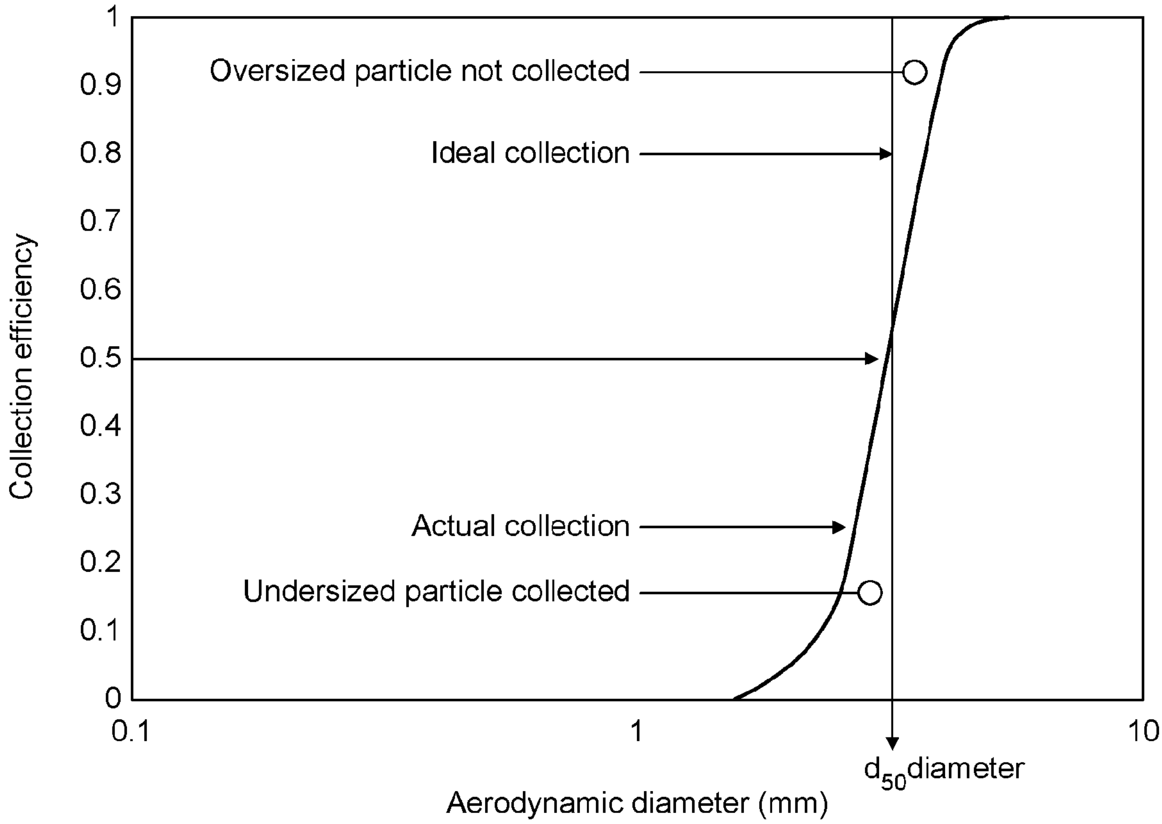
1
0.9
0.8
0.7
0.6
0.5
0.4
0.3
0.2
0.1
0
Collection efficiency
Oversized particle not collected
Ideal collection
Actual collection
Undersized particle collected
0.1
1
10
$d_{50}$diameter
Aerodynamic diameter (mm)

FIG. 2

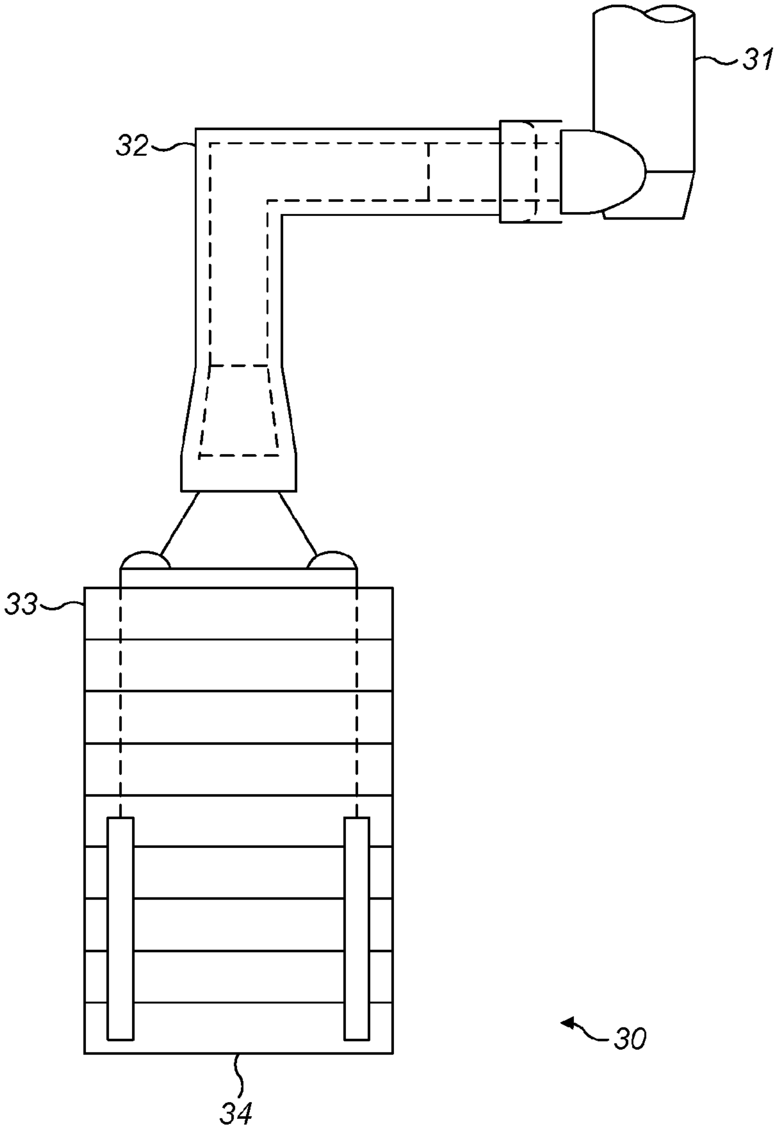


FIG. 3

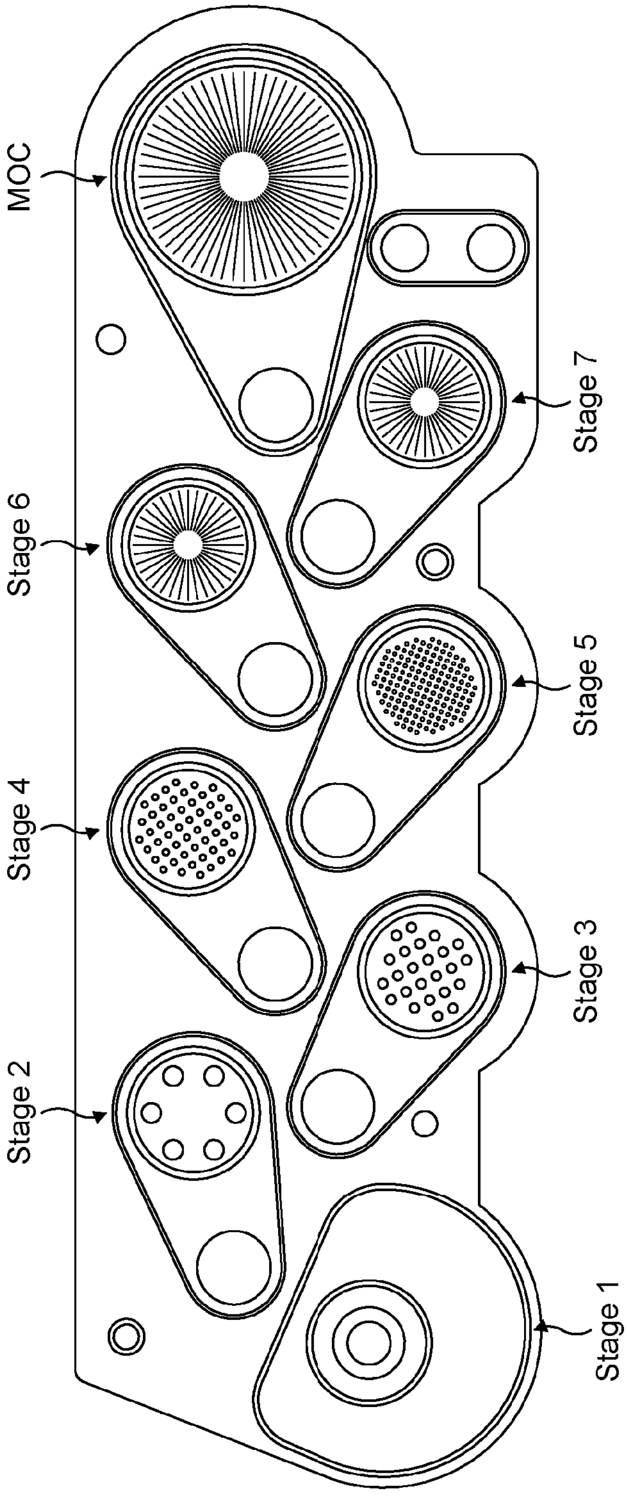


FIG. 4A

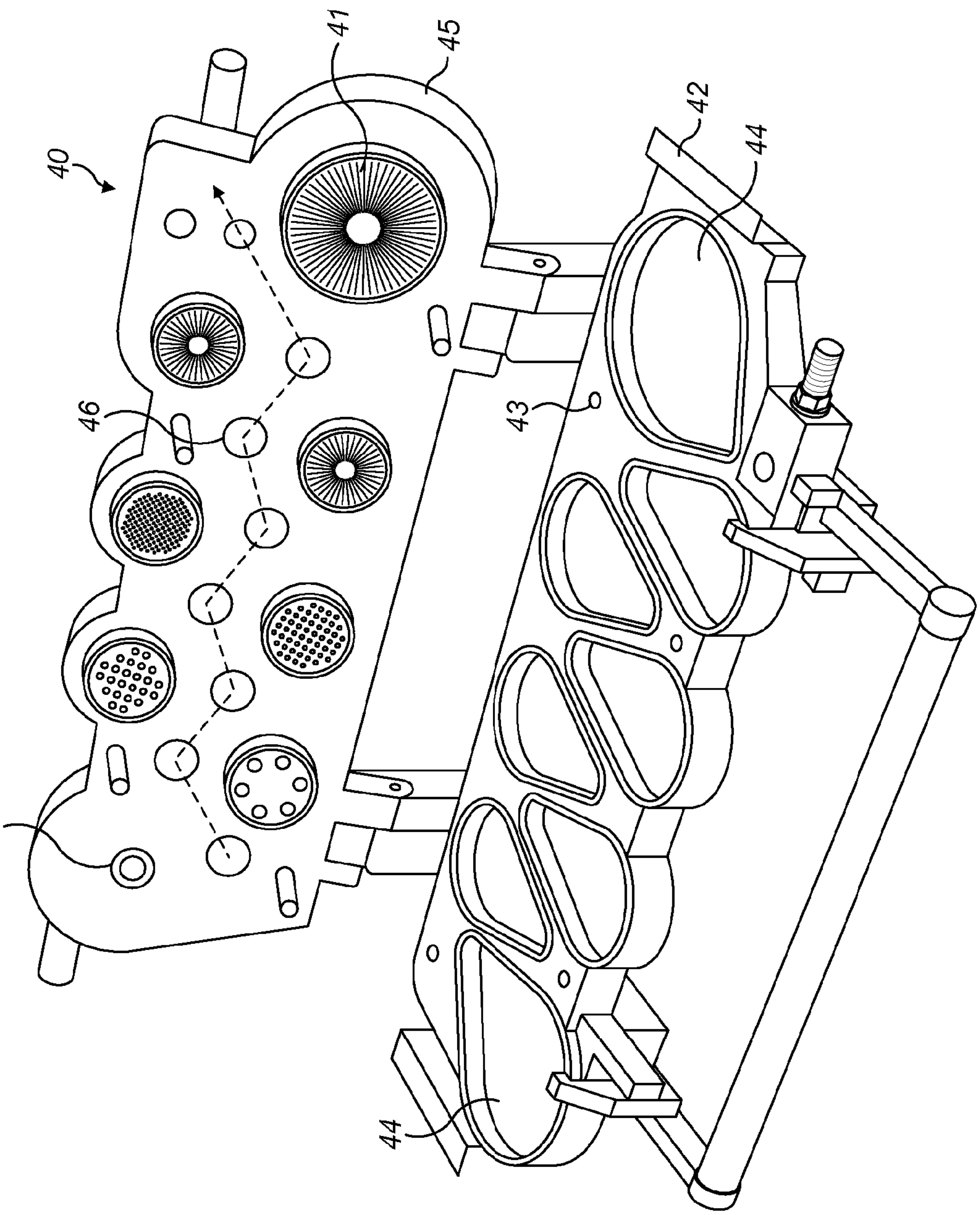


FIG. 4B

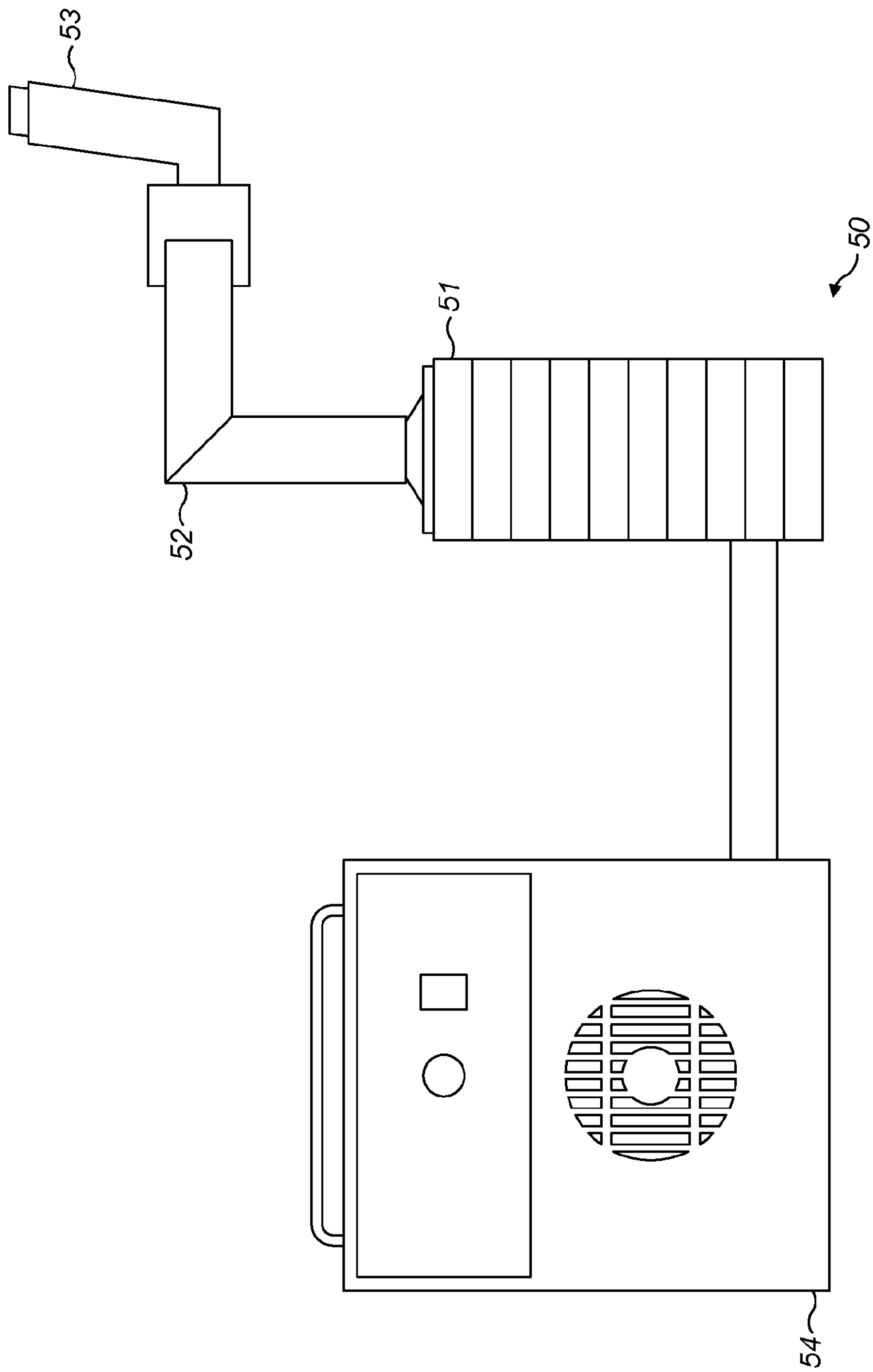


FIG. 5

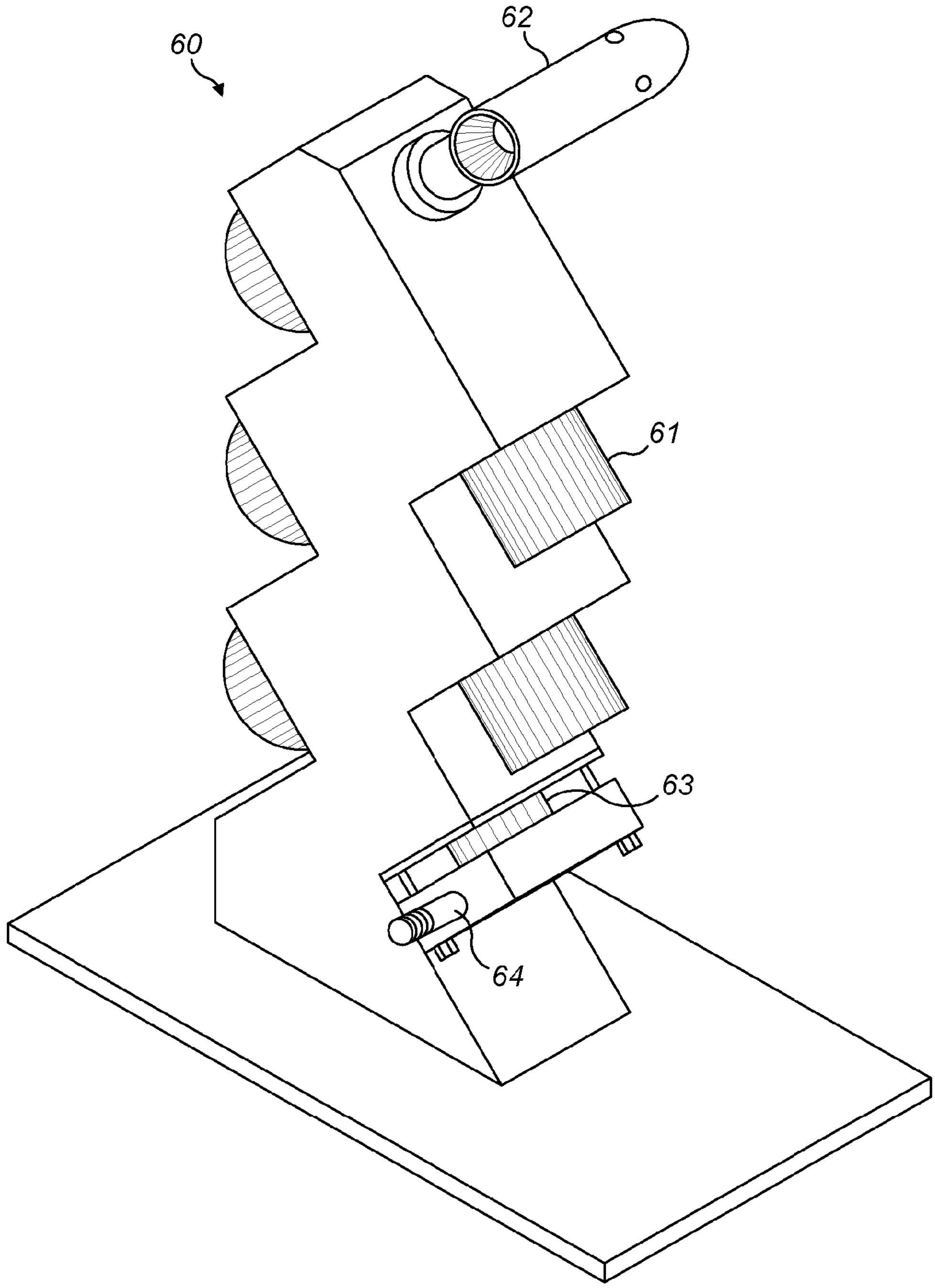


FIG. 6

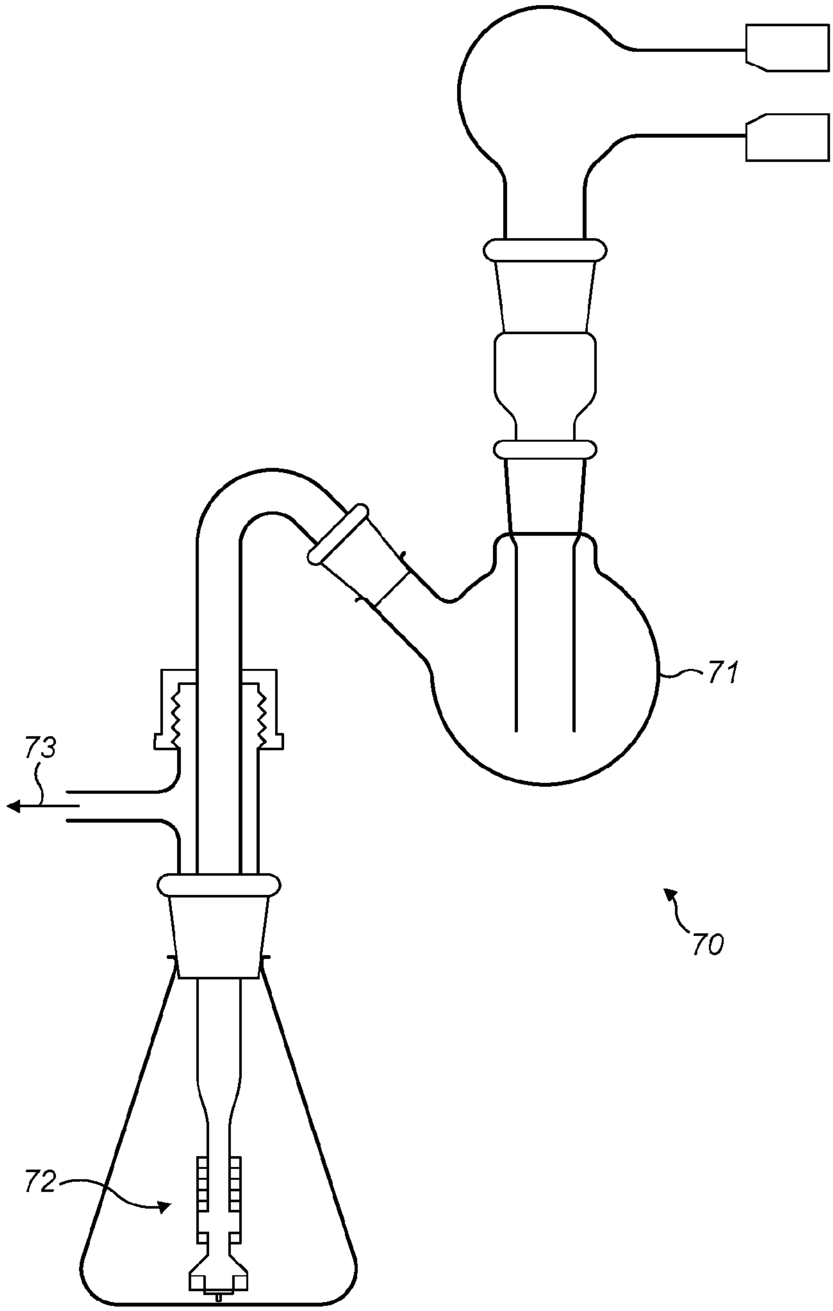


FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 20070122349 A1

### Non-patent literature cited in the description

- **MARK COPLEY.** *Understanding cascade impaction and its importance for inhaler testing,* July 2007, www.copleyscientific.com
- Copley Scientific, Quality Solutions for Inhaler Testing Brochure. 2015